(19) Europäisches Patentamt
European Patent Office
Office européen des brevets

(11) Veröffentlichungsnummer: **0 292 820 B1**

# (12) EUROPÄISCHE PATENTSCHRIFT

(45) Veröffentlichungstag der Patentschrift: **22.07.92**

(51) Int. Cl.5: **C07D 207/06**, C07D 207/08, A61K 31/40

(21) Anmeldenummer: **88107783.8**

(22) Anmeldetag: **14.05.88**

Die Akte enthält technische Angaben, die nach dem Eingang der Anmeldung eingereicht wurden und die nicht in dieser Patentschrift enthalten sind.

## (54) 3-Phenyl-2-styryl-pyrrolidine, ihre Herstellung und Verwendung.

(30) Priorität: **23.05.87 DE 3717394**

(43) Veröffentlichungstag der Anmeldung:
**30.11.88 Patentblatt 88/48**

(45) Bekanntmachung des Hinweises auf die Patenterteilung:
**22.07.92 Patentblatt 92/30**

(84) Benannte Vertragsstaaten:
**AT BE CH DE ES FR GB IT LI NL**

(56) Entgegenhaltungen:
**EP-A- 0 219 055**
**DE-A- 3 632 589**
**US-A- 4 548 951**

**CHEMICAL ABSTRACTS, Band 98, Nr. 11, 14. 03. 1983, Seite 590, Zusammenfassung-Nr. 89 708f, Columbus, Ohio, US; HERBERT, R.B. et al.: "Synthesis of ( = )-norruspoline"**

**CHEMICAL ABSTRACTS, Band 107, Nr. 25, 21. 12. 1987, Seite 735, Zusammenfassung-Nr. 236 157k, Columbus, Ohio, US; KAZARYAN, A. TS. et al.: "Reaction of N-(2,2-dimethyl-3-phenylpropylidene)benzylamine with sodium."**

(73) Patentinhaber: **BASF Aktiengesellschaft**
**Carl-Bosch-Strasse 38**
**W-6700 Ludwigshafen(DE)**

(72) Erfinder: **Himmele, Walter, Dr.**
**Eichenweg 14**
**W-6909 Walldorf(DE)**
Erfinder: **Steiner, Gerd, Dr.**
**Oberer Waldweg 1**
**W-6719 Kirchheim(DE)**
Erfinder: **Teschendorf, Hans-Juergen, Dr.**
**Georg-Nuss-Strasse 5**
**W-6724 Dudenhofen(DE)**
Erfinder: **Weifenbach, Harald, Dr.**
**Londoner Ring 71**
**W-6700 Ludwigshafen(DE)**

EP 0 292 820 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 3-Phenyl-2-styryl-pyrrolidine, ein Verfahren zu ihrer Herstellung sowie deren Verwendung zur Bekämpfung von Krankheiten.

Es sind bereits eine Reihe von Wirkstoffen bekannt, die als Sedativa und Tranquilizer zur Behandlung von psychischen Störungen eingesetzt werden (vgl. Rote Liste 1985, Indikationsgruppen 48 81 und 70 84).

Es wurde nun gefunden, daß sich eine neue Klasse von Verbindungen, nämlich 3-Phenyl-2-styryl-pyrrolidine der Formel I

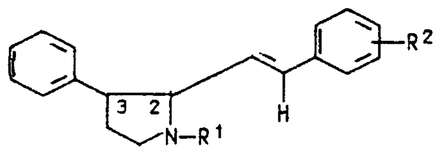

I,

worin
$R^1$ Wasserstoff oder $C_1$-$C_6$-Alkyl und
$R^2$ Wasserstoff oder Halogen
bedeuten, und ihre Salze mit physiologisch verträglichen Säuren, gut zur Behandlung von psychischen Störungen eignen.

Von den Verbindungen der Formel I sind diejenigen bevorzugt, in denen
$R^1$ Wasserstoff, Methyl, Ethyl oder n-Propyl, und
$R^2$ Wasserstoff, Fluor oder Chlor bedeuten, sowie solche, in denen $R^2$ Wasserstoff ist.

Die neuen Verbindungen können als cis- und/oder trans-Isomere sowie als Racemate oder optische Antipoden vorliegen:

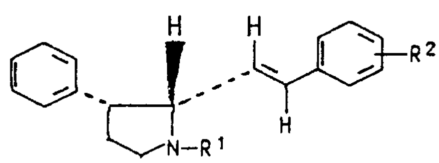

cis

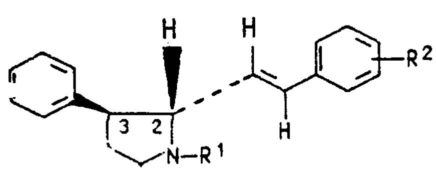

trans

Insbesondere sind folgende Verbindungen zu nennen:
cis-3-Phenyl-2-trans-styryl-N-methyl-pyrrolidin
trans-3-Phenyl-2-trans-styryl-N-methyl-pyrrolidin
cis-3-Phenyl-2-trans-styryl-N-ethyl-pyrrolidin
trans-3-Phenyl-2-trans-styryl-N-ethyl-pyrrolidin
cis-3-Phenyl-2-trans-styryl-N-n-propyl-pyrrolidin und

trans-3-Phenyl-2-trans-styryl-N-n-propyl-pyrrolidin

Die neuen Verbindungen lassen sich herstellen, indem man

a) eine Verbindung der Formel II

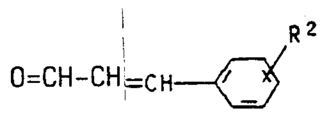

II,

worin $R^2$ die angegebene Bedeutung besitzt, und eine Verbindung der Formel III

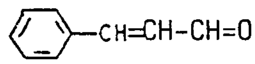

III,

zusammen mit einem Amin der Formel IV

$R^1NH_2$ IV,

worin $R^1$ die angegebene Bedeutung besitzt, in Gegenwart eines Katalysators auf der Basis eines Metalles aus der Eisengruppe bei 70 - 150 ° C hydriert oder

b) eine Verbindung der Formel V

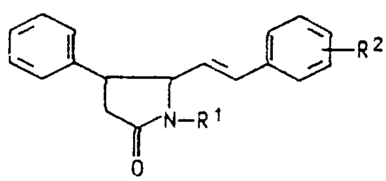

V,

worin $R^1$ und $R^2$ die angegebene Bedeutung besitzen, reduziert, und die so erhaltenen Verbindungen gegebenenfalls in ihre Salze mit physiologisch verträglichen Säuren überführt.

Bei dem Verfahren a) reagieren 1 Mol Verbindung II, 1 Mol Verbindung III mit 1 Mol Amin IV unter Aufnahme von 2 Mol Wasserstoff.

Die Umsetzung wird zwischen 70 und 150 ° C, vorzugsweise bei 80 - 110 ° C und insbesondere bei 85 - 100 ° C durchgeführt. Die Hydrierung gelingt bei einem Druck von 30 - 250 bar Wasserstoff, wobei im Bereich von 120 - 180 bar die besten Ausbeuten erzielt werden.

Als Katalysator eignet sich für die Hydrierung ein Metall aus der Eisengruppe, insbesondere Kobalt oder Nickel. Besonders bewährt hat sich ein Kobalt vom Raney-Typ, das durch Lagern bzw. Tempern oder durch leichte Beeinflussung mit Luft partiell inaktiviert wurde. In den Beispielen wurde als Katalysator für die Umsetzung der $\alpha,\beta$-ungesättigten Carbonylverbindungen ein Raney-Kobalt verwendet, dessen Reaktivität durch 200 - 600 stündige Lagerung unter Wasser bei Temperaturen von 70 - 90 ° C abgesenkt worden war. Für die Umsetzung ist auch Raney-Nickel geeignet, dessen Hydrieraktivität verringert worden ist. Wird die Inaktivierung des Katalysators zu weit getrieben, dann entstehen bei der Hydrierung in erster Linie höhersiedende Kondensationsprodukte. Ist der Katalysator zu aktiv, werden insbesondere die regulären Reaktionsprodukte der aminierenden Hydrierung erhalten.

Die Reaktion wird in einem Lösungsmittel wie Ethanol oder Tetrahydrofuran in einem Autoklaven durchgeführt. Die Verbindungen der Formel I werden am einfachsten durch fraktionierte Destillation gereinigt.

Die Reduktion b) wird durchgeführt mit Hilfe eines komplexen Metallhydrids, vorzugsweise Lithiumaluminiumhydrid in einem inerten organischen Lösungsmittel, vorzugsweise Tetrahydrofuran, bei Temperaturen zwischen 0 und 80 ° C.

Die Ausgangsverbindungen der Formel V lassen sich ferner aus 1-Alkyl-4-phenyl-5-formyl-pyrrolidin-2-on-Derivaten (DE-OS 35 37 075, 36 32 589) durch Wittig-Reaktion mit Benzylphosphonium-Salzen bzw. Benzylphosphonaten in Gegenwart von Basen herstellen.

Die bei den Umsetzungen eventuell anfallenden Diastereomerengemische der Formel I können in die reinen Diastereomeren mit cis- bzw. trans-Konfiguration aufgetrennt werden, vorzugsweise durch fraktionierte Kristallisation aus einem niederen Alkohol. Die reinen Diastereomeren lassen sich gewünschtenfalls in die entsprechenden Antipoden aufspalten, z.B. über die klassische Racematspaltung durch Bildung diastereomerer Salze unter Einsatz optisch aktiver Säuren. Beispielsweise löst man die freie Base der Formel I in einem niederen Alkohol und gibt 1 Äquivalent (+)- oder (-)-Weinsäure, (+)- oder (-)-Dibenzoyl-Weinsäure oder (+)- bzw. (-)-Di-p-toluoyl-Weinsäure hinzu. Die fraktionierte Kristallisation liefert dann zunächst das schwerer lösliche der beiden diastereomeren Salze, das bis zum konstanten Drehwert umkristallisiert wird. Nach Freisetzung der freien Base erhält man auf diese Weise die enantiomerenreine Form der Verbindung I.

Die Zahl der Isomeren, die bei der erfindungsgemäßen Reaktion entstehen, läßt sich herabsetzen, indem man als Ausgangsstoffe II und III identische Substanzen wählt. Diese Vorgehensweise stellt eine bevorzugte Ausführungsform der Erfindung dar.

Die freien 3-Phenyl-2-styryl-pyrrolidine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure und Zitronensäure.

Die neuen Substanzen eignen sich für die Behandlung von psychischen Störungen, insbesondere Depressionen.

Der Wirkmechanismus einer Gruppe von therapeutisch häufig eingesetzten Antidepressiva (z.B. tricyclischer Antidepressiva) umfaßt die Hemmung der neuronalen Aufnahme von Transmittersubstanzen (Norepinephrin, Dopamin, Serotonin). Diese Eigenschaft wird in biologischen Testmodellen zur Charakterisierung von potentiellen Antidepressiva benutzt.

Die folgende Methode wurde angewendet:

Hemmung der Neurotransmitter-Aufnahme in Rattengehirn-Synaptosomen

Hippocampus, Corpus striatum und Cortex von Rattengehirn wurden präpariert und in 0,32 M Saccharose-Lösung homogenisiert. Durch Differential-Zentrifugierenerhielt man Synaptosome, die in Pufferlösung supendiert wurden. Die Synaptosome haben die Fähigkeit, zugesetzte Neurotransmitter-Substanzen (z. B. Norepinephrin, Dopamin, Serotonin) aus dem umgebenden Medium aktiv aufzunehmen. Mit Hilfe von Aufnahmeinhibitoren ist es möglich, diesem Vorgang in Abhängigkeit von der Konzentration entgegenzuwirken. Die Synaptosome wurden mit den Testsubstanzen in verschiedenen Konzentrationen gemischt und dann bei 37 ° C mit 3 H-Norepinephrin (Hippocampus), 3 H-Dopamin (Corpus striatum) und 3 H-Serotonin (Cortex) bebrütet. Die Substratkonzentration betrug etwa 10 nM. Die Aufnahme wurde durch Verdünnung mit eiskalter Pufferlösung bestimmt; danach wurden die Synaptosome durch Zentrifugieren abgetrennt und die 3 H-Aktivität im Sediment gemessen. Ein Blindwert wurde durch Bebrüten bei 0 ° C bestimmt.

Die mittlere Hemmkonzentration (IC 50) wurde aus den für die verschiedenen Inhibitorkonzentrationen im Vergleich zum Blindversuch gefundenen Hemmwerten durch lineare Regression nach logit-log-Umwandlung errechnet.

| Substanzen nach Beispiel Nr. | Hemmung der Neurotransmitter-Aufnahme in Synaptosomen | | |
|---|---|---|---|
| | Norepinephrin IC 50 µM/l | Dopamin IC 50 µM/l | Serotonin IC 50 µM/l |
| 1 cis | 0,029 | 0,12 | >1,0 |
| 1 trans | 0,0073 | 0,092 | >1,0 |
| 2 cis | 0,043 | 0,18 | >1,0 |
| 2 trans | 0,0055 | 0,023 | >1,0 |
| 3 cis | ~0,4 | ~1,0 | >1,0 |
| trans | 0,16 | 0,4 | >1,0 |
| Imipramin | 0,014 | >1,0 | 0,13 |

Die erfindungsgemäßen Verbindungen zeichnen sich dadurch aus, daß sie sowohl den Uptake von

Noradrenalin als auch den Uptake von Dopamin hemmen, die Serotoninaufnahme wird dagegen nicht beeinflußt. Die Substanzen unterscheiden sich somit im Profil von Imipramin, einem Prototyp eines klinisch gebräuchlichen Antidepressivums. Von der Wirkstärke her wird die Noradrenalinaufnahmehemmung von Imipramin durch die erfindungsgemäßen Verbindungen teilweise deutlich übertroffen (Beispiel 1 trans, 2 trans). Die Hemmung des Dopaminuptakes ist eine zusätzliche Wirkungskomponente, die Imipramin nicht besitzt.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 20 mg/kg Körpergewicht bei oraler Gabe und zwischen 0,1 und 2 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sukker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.%.

Beispiel 1

Cis- und trans-3-Phenyl-2-trans-styryl-N-methylpyrrolidin

In einen 10-1-Rührautoklaven wurden 1,4 kg Monomethylamin, 1,6 kg Ethanol und 0,1 kg Raney-Kobalt gegeben. Der Autoklav wurde auf 100°C erhitzt und unter einem Druck von 150 bar Wasserstoff zugeführt. Im Verlauf von 10 h wurden 1,6 kg Zimtaldehyd zugepumpt. Nach Ende der Wasserstoffaufnahme wurde das Reaktionsgemisch nach dem Abkühlen filtriert und fraktioniert destilliert. Die bei 153 - 184°C/2 mbar übergehende Fraktion enthielt 99,5 g cis-3-Phenyl-2-trans-styryl-N-methyl-pyrrolidin und 68,5 g trans-3-Phenyl-2-trans-styryl-N-methyl-pyrrolidin.

Das so erhaltene Diastereomerengemisch wurde durch fraktionierte Destillation über eine Füllkörperkolonne mit einer Edelstahldrahtnetzwendel (Länge 160 cm) getrennt. Das cis-Diastereomere ging bei 157°C /2 mbar über und war zu über 95 % rein (Schmp. des Hydrochlorids 190 - 191°C), das trans-Diastereomere ging bei 160 - 161°C/2 mbar über und war zu über 90 % rein (Schmp. des Hydrochlorids 202 - 204°C).

Beispiel 2

Cis- und trans-3-Phenyl-2-trans-styryl-N-ethyl-pyrrolidin

In einem 10-1-Autoklaven wurden 2,0 kg Zimtaldehyd mit 1,4 kg Ethylamin bei 90°C unter einem Wasserstoffdruck von 150 bar in Gegenwart von 100 g Raney-Kobalt in 1,6 kg Ethanol umgesetzt. Die Zeit für das Zupumpen des Zimtaldehyds betrag 10 h. Nach Zugabe des Zimtaldehyds wurden die Reaktionsbedingungen noch 6 h aufrecht erhalten.

Das Reaktionsgemisch wurde anschließend fraktioniert destilliert. Bei 160 - 165 °C/2 mbar wurde eine Fraktion erhalten, die 141 g cis- und 161 g trans-3-Phenyl-2-trans-styryl-N-ethyl-pyrrolidin enthielt. Die Diastereomerentrennung erfolgte analog Beispiel 1. Schmp.: cis-Hydrobromid 220 - 223°C, trans-Hydrochlorid 144 - 146°C).

Beispiel 3

Cis- und trans-3-Phenyl-2-trans-styryl-N-n-propyl-pyrrolidin

In einem 10 1-Autoklaven wurden 2 kg Zimtaldehyd mit 1,6 kg n-Propylamin bei 90°C unter einem

Wasserstoffdruck von 150 bar in Gegenwart von 100 g Raney-Kobalt umgesetzt. Die Zeit für das Zupumpen des Zimtaldehyds betrug 10 h. Das Reaktionsgemisch wurde fraktioniert destilliert. Die bei 150 - 200 °C/2 mbar übergehende Fraktion wurde analog Beispiel 1) fraktioniert destilliert. Man erhielt bei 164 - 166°C/2 mbar cis-3-Phenyl-2-trans-styryl-N-n-propyl-pyrrolidin (85 % rein) und bei 167 - 168°C/2 mbar trans-3-Phenyl-2-trans-styryl-N-n-propyl-pyrrolidin (94 % rein).

Beispiel 4

Cis-1-Methyl-3-phenyl-2-trans-m-chlorstyryl-pyrrolidin

a) Herstellung des Ausgangsmaterials

Zu 36,0 g (77 mM) Triphenyl-m-chlor-benzyl-phosphonium-bromid in 150 ml Toluol tropfte man unter Eiskühlungs- und Stickstoffschutzgas innerhalb 20 Min. 32,8 g (77 mM) n-Butyllithium (15% in Hexan) und ließ die organgefarbene Suspension noch 15 Min. nachrühren. Anschließend tropfte man 15,6 g (77 mM) cis-5-Formyl-1-methyl-4-phenyl-pyrrolidin-2-on (DE-OS 35 37 075, 36 32 589; J. Org. Chem. 52, 4352 (1987)) in 120 ml Toluol hinzu und ließ 3 h bei Raumtemperatur nachrühren (Entfärbung nach hellgelb). Man fügte zum Ansatz 200 ml $H_2O$ hinzu, säuerte mit verdünnter HCl an, trennte nach Absaugen die Phasen, extrahierte die wäßrige Phase noch zweimal mit Toluol, wusch die vereinigten organischen Phasen mit $H_2O$, trocknete und engte ein. Das Rohprodukt wurde in 100 ml Methyl-t-butylether aufgenommen und unter Kühlen vom ausgefallenen Triphenylphosphinoxid abgesaugt. Das Filtrat wurde eingeengt und durch Säulenchromatographie (Kieselgel, Laufmittel Methylenchlorid/Methanol 98/2 gereingt, Ausbeute 16,1 g (67%). Die Styryl-Seitenkette liegt nach dem $^1$H-NMR-Spektrum zu 90% in der trans- und zu 10% in der cis-Konformation vor.

b) Herstellung des Endprodukts

Zu 0,65 g (17 mM) Lithiumaluminiumhydrid in 30 ml Ether tropfte man bei 0 bis 5°C unter Stickstoff eine Lösung aus 3,0 g (9,6 mM) cis-1-Methyl-4-phenyl-5-m-chlorstyryl-pyrrolidin-2-on in 40 ml Ether hinzu und ließ 1 Stunde bei Eiskühlung nachrühren. Anschließend wurde unter Kühlung 10%ige Natronlauge langsam zugetropft, bis der Niederschlag an der Gefäßwand konglomerierte. Die überstehende Etherphase wurde mit $H_2O$ gewaschen (pH = 10), getrocknet und eingeengt. Reinigung durch Säulenchromatographie (Kieselgel, Laufmittel Methylenchlorid/Methanol 95/5) lieferte cis-1-Methyl-3-phenyl-2-trans-m-chlorstyryl-pyrrolidin als freie Base (2,3 g, 80%). Diese wurde in Essigester aufgenommen und mit etherischer HCl in das Hydrochlorid überführt, Schmp. 53-56°C.

In analoger Weise erhielt man das trans-Diastereomere durch Einsatz von trans-5-Formyl-1-methyl-4-phenyl-pyrrolidin-2-on.

Cis- und trans-3-Phenyl-2-trans-o-chlorstyryl-N-methyl-pyrrolidin
Cis- und trans-3-Phenyl-2-trans-m-chlorstyryl-N-methyl-pyrrolidin
Cis- und trans-3-Phenyl-2-trans-p-chlorstyryl-N-methyl-pyrrolidin
Cis- und trans-3-Phenyl-2-trans-m-fluorstyryl-N-methyl-pyrrolidin
Cis- und trans-3-Phenyl-2-trans-p-fluorstyryl-N-methyl-pyrrolidin
Cis- und trans-3-Phenyl-2-trans-o-fluorstyryl-N-methyl-pyrrolidin

Beispiel 11

Auf einer Tablettenpresse werden in üblicher Weise Tabletten folgender Zusammensetzung gepreßt:

| | |
|---|---|
| 12,5 mg | Substanz des Beispiels 1 (cis) |
| 120 mg | Maisstärke |
| 13,5 mg | Gelatine |
| 45 mg | Milchzucker |
| 2,25 mg | Aerosil$^R$ (chemisch reine Kieselsäure in submikroskopischer feiner Verteilung) |
| 6,75 mg | Kartoffelstärke (als 6%iger Kleister) |

Beispiel 12

In üblicher Weise werden Dragees folgender Zusammensetzung hergestellt:

| | |
|---|---|
| 7,5 mg | Substanz des Beispiels 1 (trans) |
| 60 mg | Kernmasse |
| 60 mg | Verzuckerungsmasse |

Die Kernmasse besteht aus 9 Teilen Maisstärke, 3 Teilen Milchzucker und 1 Teil Luviskol[R] VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 13

1 g Substanz des Beispiels 2 (cis) werden als Hydrochlorid in 5000 ml Wasser unter Zusatz von NaCl gelöst und mit 0,1 H NaOH auf pH 6,0 eingestellt, so daß eine blutisotonische Lösung entsteht. Jeweils 5 ml dieser Lösung werden in Ampullen gefüllt und sterilisiert.

**Patentansprüche**

**Patentansprüche für folgende Vertragsstaaten : AT, BE, CH, DE, FR, GB, IT, LI, NL**

**1.** 3-Phenyl-2-styryl-pyrrolidine der Formel I

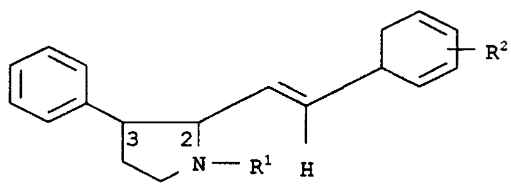

worin
$R^1$ Wasserstoff oder $C_1$-$C_6$-Alkyl und
$R^2$ Wasserstoff oder Halogen
bedeuten, und ihre Salze mit physiologisch verträglichen Säuren.

**2.** Verbindungen der Formel I gemäß Anspruch 1, in denen $R^1$ Wasserstoff, Methyl, Ethyl oder n-Propyl und $R^2$ Wasserstoff, Fluor oder Chlor bedeuten.

**3.** Verbindungen gemäß Anspruch 2, in denen $R^2$ Wasserstoff bedeutet.

**4.** Verbindung gemäß Anspruch 1, dadurch gekennzeichnet, daß sie cis-3-Phenyl-2-trans-styryl-N-methyl-pyrrolidin ist.

**5.** Verbindung gemäß Anspruch 1, dadurch gekennzeichnet, daß sie trans-3-Phenyl-2-trans-styryl-N-methyl-pyrrolidin ist.

**6.** Verbindung gemäß Anspruch 1, dadurch gekennzeichnet, daß sie cis-3-Phenyl-2-trans-styryl-N-ethyl-pyrrolidin ist.

**7.** Verbindung gemäß Anspruch 1, dadurch gekennzeichnet, daß sie trans-3-Phenyl-2-trans-styryl-N-ethyl-pyrrolidin ist.

**8.** Verbindung gemäß Anspruch 1, dadurch gekennzeichnet, daß sie cis-3-Phenyl-2-trans-styryl-N-n-propyl-pyrrolidin ist.

**9.** Verbindung gemäß Anspruch 1, dadurch gekennzeichnet, daß sie trans-3-Phenyl-2-trans-styryl-N-n-propyl-pyrrolidin ist.

**10.** Verfahren zur Herstellung der 3-Phenyl-2-styryl-pyrrolidine der Formel I gemäß Anspruch 1, dadurch gekennzeichnet, daß man

a) eine Verbindung der Formel II

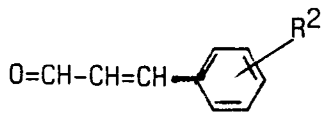

II,

worin $R^2$ die angegebene Bedeutung besitzt, und eine Verbindung der Formel III

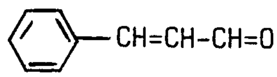

III,

zusammen mit einem Amin der Formel IV

$R^1NH_2$ IV,

worin $R^1$ die angegebene Bedeutung besitzt, in Gegenwart eines Katalysators auf der Basis eines Metalles aus der Eisengruppe bei 70 - 150 °C hydriert oder

b) eine Verbindung der Formel V

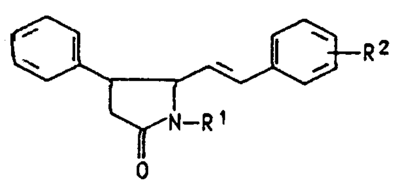

V,

worin $R^1$ und $R^2$ die angegebene Bedeutung besitzen, reduziert, und die so erhaltenen Verbindungen gegebenenfalls in ihre Salze mit physiologisch verträglichen Säuren überführt.

**11.** Therapeutisches Mittel enthaltend eine Verbindung der Formel I gemäß Anspruch 1.

**Patentansprüche für folgenden Vertragsstaat : ES**

**1.** Verfahren zur Herstellung der 3-Phenyl-2-styryl-pyrrolidine der Formel I

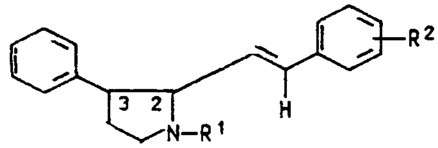

I,

worin
$R^1$ Wasserstoff oder $C_1$-$C_6$-Alkyl und
$R^2$ Wasserstoff oder Halogen

bedeuten, und deren Salzen mit physiologisch verträglichen Säuren, dadurch gekennzeichnet, daß man
a) eine Verbindung der Formel II

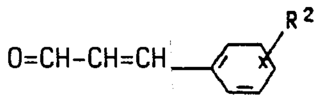

II,

worin $R^2$ die angegebene Bedeutung besitzt, und eine Verbindung der Formel III

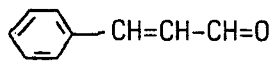

III,

zusammen mit einem Amin der Formel IV

$R^1NH_2$ IV,

worin $R^1$ die angegebene Bedeutung besitzt, in Gegenwart eines Katalysators auf der Basis eines Metalles aus der Eisengruppe bei 70 - 150 ° C hydriert oder
b) eine Verbindung der Formel V

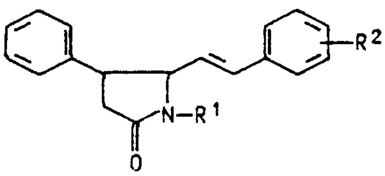

V,

worin $R^1$ und $R^2$ die angegebene Bedeutung besitzen, reduziert, und die so erhaltenen Verbindungen gegebenenfalls in ihre Salze mit physiologisch verträglichen Säuren überführt.

2. Verfahren gemäß Anspruch 1, dadurch gekennzeichnet, daß man Verbindungen herstellt, in denen $R^1$ Wasserstoff, Methyl, Ethyl oder n-Propyl und $R^2$ Wasserstoff, Fluor oder Chlor bedeuten.

3. Verfahren gemäß Anspruch 1, dadurch gekennzeichnet, daß man Verbindungen herstellt, in denen $R^2$ Wasserstoff ist.

4. Verfahren gemäß Anspruch 1, dadurch gekennzeichnet, daß man cis-3-Phenyl-2-trans-styryl-N-methyl-pyrrolidin herstellt.

5. Verfahren gemäß Anspruch 1, dadurch gekennzeichnet, daß man trans-3-Phenyl-2-trans-styryl-N-methyl-pyrrolidin herstellt.

6. Verfahren gemäß Anspruch 1, dadurch gekennzeichnet, daß man cis-3-Phenyl-2-trans-styryl-N-ethyl-pyrrolidin herstellt.

7. Verfahren gemäß Anspruch 1, dadurch gekennzeichnet, daß man trans-3-Phenyl-2-trans-styryl-N-ethyl-pyrrolidin herstellt.

8. Verfahren gemäß Anspruch 1, dadurch gekennzeichnet, daß man cis-3-Phenyl-2-trans-styryl-N-n-propyl-pyrrolidin herstellt.

9. Verfahren gemäß Anspruch 1, dadurch gekennzeichnet, daß man trans-3-Phenyl-2-trans-styryl-N-n-propyl-pyrrolidin herstellt.

**Claims**
**Claims for the following Contracting States : AT, BE, CH, DE, FR, GB, IT, LI, NL**

1. A 3-phenyl-2-styrylpyrrolidine of the formula I

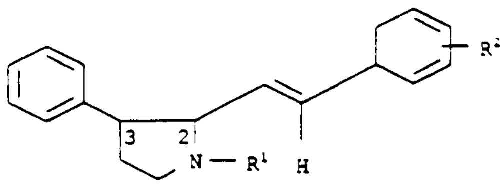

where $R^1$ is hydrogen or $C_1$-$C_6$-alkyl, and $R^2$ is hydrogen or halogen, or a salt thereof with a physiologically tolerated acid.

2. A compound of the formula I as claimed in claim 1 where $R^1$ is hydrogen, methyl, ethyl or n-propyl, and $R^2$ is hydrogen, fluorine or chlorine.

3. A compound as claimed in claim 2 where $R^2$ is hydrogen.

4. A compound as claimed in claim 1, which is cis-3-phenyl-2-trans-styryl-N-methylpyrrolidine.

5. A compound as claimed in claim 1, which is trans-3-phenyl-2-trans-styryl-N-methylpyrrolidine.

6. A compound as claimed in claim 1, which is cis-3-phenyl-2-trans-styryl-N-ethylpyrrolidine.

7. A compound as claimed in claim 1, which is trans-3-phenyl-2-trans-styryl-N-ethylpyrrolidine.

8. A compound as claimed in claim 1, which is cis-3-phenyl-2-trans-styryl-N-n-propylpyrrolidine.

9. A compound as claimed in claim 1, which is trans-3-phenyl-2-trans-styryl-N-n-propylpyrrolidine.

10. A process for preparing a 3-phenyl-2-styrylpyrrolidine of the formula I as claimed in claim 1, which comprises
   a) hydrogenating a compound of the formula II

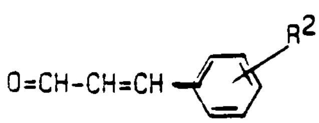

II

where $R^2$ is as defined above, and a compound of the formula III

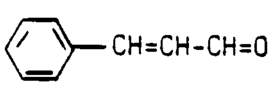

III

together with an amine of the formula IV

$R^1NH_2$ IV

where $R^1$ is as defined above, in the presence of a catalyst based on a metal of the iron group at 70 - 150 ° C or

b) reducing a compound of the formula V

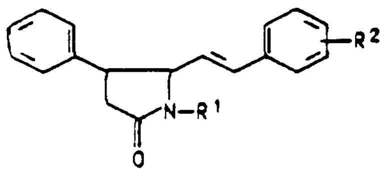

V

where $R^1$ and $R^2$ are each as defined above, and if desired converting the compound thus obtained into a salt with a physiologically tolerated acid.

**11.** A therapeutic composition containing a compound of the formula I as claimed in claim 1.

**Claims for the following Contracting State : ES**

**1.** A process for preparing a 3-phenyl-2-styrylpyrrolidine of the formula I

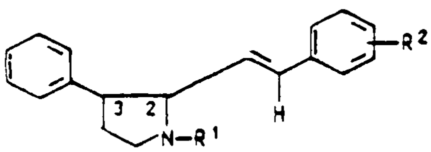

I

where $R^1$ is hydrogen or $C_1$-$C_6$-alkyl, and $R^2$ is hydrogen or halogen, or a salt thereof with a physiologically tolerated acid, which comprises

a) hydrogenating a compound of the formula II

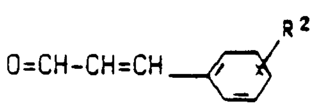

II

where $R^2$ is as defined above, and a compound of the formula III

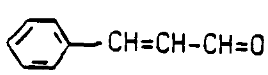

III

together with an amine of the formula IV

$R^1NH_2$ IV

where $R^1$ is as defined above, in the presence of a catalyst based on a metal of the iron group at 70 - 150 ° C or

b) reducing a compound of the formula V

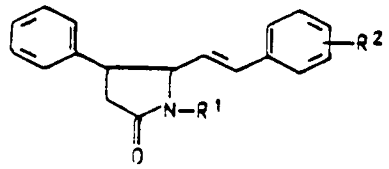

V

where $R^1$ and $R^2$ are each as defined above, and if desired converting the compound thus obtained into a salt with a physiologically tolerated acid.

**2.** A process as claimed in claim 1 wherein, in the compound prepared, $R^1$ is hydrogen, methyl, ethyl or n-propyl, and $R^2$ is hydrogen, fluorine or chlorine.

**3.** A process as claimed in claim 1, wherein, in the compound prepared, $R^2$ is hydrogen.

**4.** A process as claimed in claim 1, wherein cis-3-phenyl-2-trans-styryl-N-methylpyrrolidine is prepared.

**5.** A process as claimed in claim 1, wherein trans-3-phenyl-2-trans-styryl-N-methylpyrrolidine is prepared.

**6.** A process as claimed in claim 1, wherein cis-3-phenyl-2-trans-styryl-N-ethylpyrrolidine is prepared.

**7.** A process as claimed in claim 1, wherein trans-3-phenyl-2-trans-styryl-N-ethylpyrrolidine is prepared.

**8.** A process as claimed in claim 1, wherein cis-3-phenyl-2-trans-styryl-N-n-propylpyrrolidine is prepared.

**9.** A process as claimed in claim 1, wherein trans-3-phenyl-2-trans-styryl-N-n-propylpyrrolidine is prepared.

**Revendications**
**Revendications pour les Etats contractants suivants : AT, BE, CH, DE, FR, GB, IT, LI, NL**

**1.** 3-phényl-2-styryl-pyrrolidines de formule I

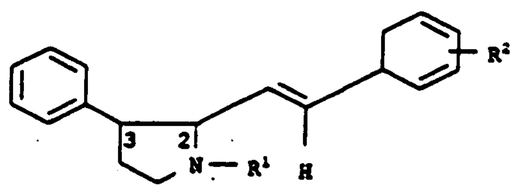

dans laquelle
$R^1$ représente l'hydrogène ou un groupe alkyle en C1-C6, et
$R^2$ représente l'hydrogène ou un halogène,
et leurs sels d'acides acceptables pour l'usage pharmaceutique.

**2.** Composés de formule I de la revendication 1, dans laquelle $R^1$ représente l'hydrogène, un groupe méthyle, éthyle ou n-propyle et $R^2$ l'hydrogène, le fluor ou le chlore.

**3.** Composés selon la revendication 2, dans lesquels $R^2$ représente l'hydrogène.

**4.** Composé selon revendication 1, caractérisé en ce qu'il consiste en la cis-3-phényl-2-trans-styryl-N-méthyl-pyrrolidine.

**5.** Composé selon revendication 1, caractérisé en ce qu'il consiste en la trans-3-phényl-2-trans-styryl-N-méthyl-pyrrolidine.

**6.** Composé selon revendication 1, caractérisé en ce qu'il consiste en la cis-3-phényl-2-trans-styryl-N-éthyl-pyrrolidine.

**7.** Composé selon revendication 1, caractérisé en ce qu'il consiste en la trans-3-phényl-2-trans-styryl-N-éthyl-pyrrolidine.

**8.** Composé selon revendication 1, caractérisé en ce qu'il consiste en la cis-3-phényl-2-trans-styryl-N-n-propyl-pyrrolidine.

**9.** Composé selon revendication 1, caractérisé en ce qu'il consiste en la trans-3-phényl-2-trans-styryl-N-n-propyl-pyrrolidine.

**10.** Procédé de préparation des 3-phényl-2-styryl-pyrrolidines de formule I de la revendication 1, caractérisé en ce que :

a) on hydrogène à une température de 70 à 150 degrés C un composé de formule II

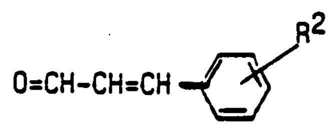

II,

dans laquelle $R^2$ a les significations indiquées ci-dessus, et un composé de formule III

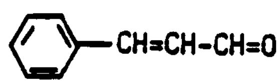

III,

avec une amine de formule IV

$R^1NH_2$ IV

dans laquelle $R^1$ a les significations indiquées ci-dessus, en présence d'un catalyseur à base d'un métal du groupe du fer, ou bien

b) on réduit un composé de formule V

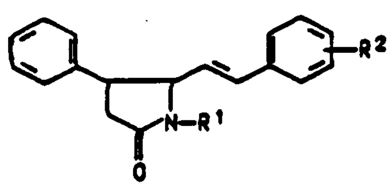

V,

dans laquelle $R^1$ et $R^2$ ont les significations indiquées ci-dessus et, le cas échéant, on convertit les composés ainsi obtenus en leurs sels d'acides acceptables pour l'usage pharmaceutique.

**11.** Agent thérapeutique contenant un composé de formule I de la revendication 1.

**Revendications pour l'Etat contractant suivant : ES**

**1.** Procédé de préparation des 3-phényl-2-styryl-pyrrolidines de formule I

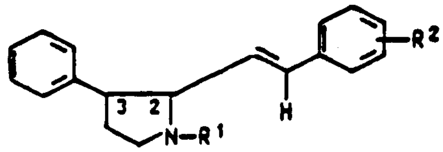

I.

dans laquelle
$R^1$ représente l'hydrogène ou un groupe alkyle en C1-C6, et
$R^2$ représente l'hydrogène ou un halogène,
et de leurs sels d'acides acceptables pour l'usage pharmaceutique,
caractérisé en ce que :

a) on hydrogène à une température de 70 à 150 degrés C un composé de formule II

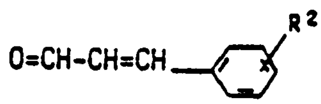

II.

dans laquelle $R^2$ a les significations indiquées ci-dessus, et un composé de formule III

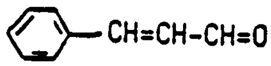

III.

avec une amine de formule IV

$R^1NH_2$ IV

dans laquelle $R^1$ a les significations indiquées ci-dessus, en présence d'un catalyseur à base d'un métal du groupe du fer, ou bien

b) on réduit un composé de formule V

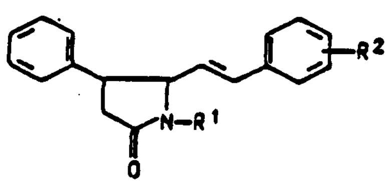

V.

dans laquelle $R^1$ et $R^2$ ont les significations indiquées ci-dessus, et le cas échéant on convertit les composés ainsi obtenus en leurs sels d'acides acceptables pour l'usage pharmaceutique.

**2.** Procédé selon la revendication 1, caractérisé en ce que l'on prépare des composés pour lesquels $R^1$ représente l'hydrogène, un groupe méthyle, éthyle ou n-propyle et $R^2$ l'hydrogène, le fluor ou le chlore.

**3.** Procédé selon la revendication 1, caractérisé en ce que l'on prépare des composés pour lesquels $R^2$ représente l'hydrogène.

**4.** Procédé selon la revendication 1, caractérisé en ce que l'on prépare la cis-3-phényl-2-trans-styryl-N-méthyl-pyrrolidine.

**5.** Procédé selon la revendication 1, caractérisé en ce que l'on prépare la trans-3-phényl-2-trans-styryl-N-méthyl-pyrrolidine.

6. Procédé selon la revendication 1, caractérisé en ce que l'on prépare la cis-3-phényl-2-trans-styryl-N-éthyl-pyrrolidine.

7. Procédé selon la revendication 1, caractérisé en ce que l'on prépare la trans-3-phényl-2-trans-styryl-N-éthyl-pyrrolidine.

8. Procédé selon la revendication 1, caractérisé en ce que l'on prépare la cis-3-phényl-2-trans-styryl-N-n-propyl-pyrrolidine.

9. Procédé selon la revendication 1, caractérisé en ce que l'on prépare la trans-3-phényl-2-trans-styryl-N-n-propyl-pyrrolidine.